# EUROPEAN PATENT SPECIFICATION

(11) **EP 4 106 692 B1**
(45) Date of publication and mention of the grant of the patent: **26.07.2023**
(21) Application number: 21708314.6
(22) Date of filing: 18.02.2021
(51) Int. Cl.: A61F 5/445, A61F 5/44

(54) **AN OUTLET VALVE FOR AN OSTOMY APPLIANCE**
AUSLASSVENTIL FÜR EINE STOMAVORRICHTUNG
SOUPAPE D'ECHAPPEMENT POUR UN APPAREIL POUR STOMIE

(30) Priority: 19.02.2020 GB 202002314; 19.02.2020 GB 202002316
(43) Date of publication of application: 28.12.2022
(73) Proprietor: ConvaTec Limited, Deeside, Flintshire CH5 2NU (GB)
(72) Inventor: HOLROYD, Simon, London Greater London W6 7HJ (GB); BAKER, Dominic, London Greater London W6 7HJ (GB); TAAL, Stefan, London Greater London W6 7HJ (GB)
(74) Representative: Wilson Gunn
(86) International application number: PCT/GB2021/050391
(87) International publication number: WO 2021/165674

(56) References cited:
- EP-A1- 0 680 296
- WO-A1-99/45866
- US-A- 5 902 294
- US-A- 6 021 928
- US-A1- 2013 327 795
- US-B2- 9 333 110

## Description

### Technical Field

The present disclosure relates to an outlet valve an ostomy appliance, particularly for a drainage of a liquid output from an ostomy bag.

### Background of the disclosure

An ostomy appliance may be provided with an outlet valve for draining liquid from the bag of the ostomy appliance, either for direct disposal of the liquid or via an additional appliance such as a drain bag which may be attached to the valve in use. There are many forms of ostomy appliance which try to provide an easily drainable appliance.

US6021928 discloses a tap for a drainage bag which can be welded to the bag material with the tap in a ready-assembled condition. The tap includes an integral attachment portion and a housing. An L-shaped tubular tap member with a handle is inserted in an aperture of the housing, and is rotatable in the aperture between a closed position in which the outlet points upwardly, and an open position in which the outlet points downwardly. In the closed position, a peripheral region of the attachment portion projects beyond the tap member on all sides, to enable the assembled tap to be welded to the bag material.

GB2285496 discloses a valve, particularly for use as an outlet valve of a medical bag, such as a urine drainage bag, which is opened and closed by relative rotation of its inlet and outlet pipes about an axis perpendicular to their longitudinal axes. The inlet and outlet pipes project from housing parts. One housing part has a barrel fitting inside the other housing part. The housing parts each have an aperture that are in alignment when the pipes are aligned. When the pipes both face the same direction the valve is closed. The apertures may take various forms. Also, a ball may be used or there may be a separate barrel interengaging with each pipe housing and having a lost motion drive therewith.

EP0680296 discloses an outlet valve consisting of a base member and at least one movable means which together form a discharge tube or hose member, a stop valve and a closing valve, said movable means being rotatably and/or linearly displaceable and by means of which the valve system can be set in three positions, a first position in which both the stop valve and the closing valve are open, a second position in which the stop valve is closed and the closing valve is open, and a third position in which both valves are closed. A collection bag provided with an outlet valve as stated above.

WO99/45866A1 discloses an outlet valve system for a urine collection bag. The system comprises a base member secured to the bag and a moveable member sealing fixed to the base member. The moveable member has a discharge tube rotatable with respect to he base member out of a plane defined by the outer surface of the collection bag. The moveable member has at least one open position and at least one secured closed position where the discharge tube is pointing upwards.

US93331 10B2 discloses a valve sealable to an associated container such as an ostomy pouch. The valve includes a valve body with a stem rotatably mounted to the valve body. The stem has a hollow central portion and a sealed upper portion. Rotation of the stem allows a stem opening to align with the valve body in an open configuration for permitting flow from the pouch through the valve, or to misalign with the valve body in a closed configuration to stop the flow through the valve.

US2013/3277795A1 discloses a plastic container with a pour spout for mounting on a container neck and having one or more closable pour openings. At least one cam projects over a neck wall of the container neck and the pour spout has a body configured for connection to the container neck and comprising receptacles corresponding to the cam(s). The cam(s) are formed on the inside wall of the container neck and the receptacles are formed on the outside wall of the body of the spout.

US5902294A discloses a valve for urine drainage applications with a valve housing for attachment to a urine bag/receptacle. A valve member is rotatable in the housing to select an open position in which liquid can flow through the valve to a closed position cutting off liquid flow. The valve member is rotated about an axis normal to the direction of liquid outflow from the valve and a manually operable tab is provided for rotating the valve member in the housing.

There remains a need for ostomy appliances with enhanced usability for ostomates, particularly in the area of ease of use, particularly when connecting the outlet valve to an additional appliance, for example a drain bag.

### Summary of the Disclosure

In this specification, the term "stomal output" refers to any gases or fluids or solids produced by an ostomate that may be secreted from the stoma or that exit the stoma.

In this specification, the term "stoma" refers to an opening in the body. Generally, the stoma is a surgical opening in the torso of the body. In some instances, the term "stoma" also refers to internal tissue, organs or portions thereof that are exposed by the opening. By way of non-limiting example, internal tissue may be selected from colon, ileum, small intestine, large intestine, jejunum, and duodenum, and combinations thereof. The internal tissue may be an end or a loop of a small or large intestine.

In this specification, the term "ostomate" refers to a subject that may have use of the ostomy appliance disclosed herein. While ostomate usually refers to a subject with a surgical opening, as used herein, "ostomate" may refer to a subject who has a stoma, regardless of whether the stoma was created by surgery or other means.

The term "user" may refer to an ostomate, or to another person assisting the ostomate, for example, with emptying of the stomal output from the cavity.

In this specification, the ostomy appliances disclosed herein may, for example, be used for managing a stoma created by an esophagostomy, a gastrostomy, a cholecystostomy, a choledochostomy, a cecostomy, a colostomy, a duodenostomy, an ileostomy, a jejunostomy, an appendicostomy, a tracheostomy, a urostomy, a nephrostomy, an ureterostomy, or a vesicostomy. The ostomy appliances disclosed herein may be used with additional devices including, but not limited to, a shunt, a catheter, a plug or a fecal management system.

Beneficially, the ostomy appliances of the present disclosure may permit an ostomate to increase the period of use of each ostomy appliance compared to prior art appliances. This may be achieved by providing means for draining the cavity of stomal output reliably and hygienically so as to increase an ostomate's confidence in reusing the ostomy appliance compared to some prior art appliances. Since the ostomate may be inclined to use each ostomy appliance of the present disclosure for longer, the total number of ostomy appliances used by the ostomate in a given time period may be reduced. This may produce an environmental benefit in reducing the amount of environmental waste produced.

In this specification locations and orientations of features may be described with reference to the ostomy appliance being "in use", "orientated as it would be in use" or similar. Such terms refer to the intended orientation of the ostomy appliance when it is adhered to a body of an ostomate with the ostomate in a standing position, irrespective of whether the ostomy appliance is currently performing such a use or the actual position of the ostomate. The terms "upper" and "lower" and related terms refer to the relative position of a part or portion of the ostomy appliance when orientated as it would be in use. For example, an apex of the ostomy appliance may be referred to as an "upper" apex in use of the ostomy appliance. In such an example, said apex will be intended to be the uppermost apex (in the vertical direction) of the ostomy appliance when attached to the body of a standing ostomate. However, the reader skilled in the art will appreciate that before attachment to the ostomate said apex may not always be the uppermost apex and in addition when attached the apex may not always be the uppermost apex if the ostomate adopts a non-standing position, for example lying down.

In this specification the term "outer" or "front" refers to the relative position of a part or portion of the ostomy appliance with reference to the body of an ostomate when the ostomy appliance is attached to the body. "Outer" or "front" refers to a position relatively further away from the body of the ostomate than a comparative position that is relatively closer to the body. Similarly, "back" refers to a position relatively closer to the body of the ostomate than a comparative position that is "outer" or "front".

In this specification the term "peripheral region" refers to a portion situated on or towards an edge of the item being referred to.

In this specification, the term "affixed" is used to refer to a connection in which teh component in question cannot be removed during normal use of the outlet valve or without damaging the component.

The invention is set out in the appended set of claims.

In some embodiments, one of the outlet element and the valve base may comprise the detent and the other of the outlet element and the valve base may comprise two or more protrusions. Accordingly in one embodiment of the invention, there is provided an outlet valve for an ostomy bag comprising: a valve base for attachment to a face of the ostomy appliance, the valve base defining a base opening for receiving a liquid output from the ostomy bag; and an outlet element configured to be rotatable relative to the valve base from a closed configuration in which discharge of the liquid received through the base opening is prevented, to an open configuration in which discharge of the liquid received through the base opening is enabled; wherein one of the outlet element and the valve base comprises a detent and the other of the outlet element and the valve base comprises two or more protrusions; and wherein the detent is configured to engage the one or more protrusions during a rotation of the outlet element such that the detent contacts and rides over one or more of the protrusions during the rotation. Thus, the valve provides tactile feedback at multiple positions of the outlet element allowing for better control of the valve.

Preferably, the valve base may comprise the detent, and the outlet element may comprise two or more protrusions. Accordingly in one embodiment of the invention, there is provided an outlet valve for an ostomy bag comprising: a valve base for attachment to a face of the ostomy appliance, the valve base defining a base opening for receiving a liquid output from the ostomy bag; and an outlet element configured to be rotatable relative to the valve base from a closed configuration in which discharge of the liquid received through the base opening is prevented, to an open configuration in which discharge of the liquid received through the base opening is enabled; wherein the valve base comprises a detent and the the outlet element comprises two or more protrusions and wherein the detent is configured to engage the one or more protrusions during a rotation of the outlet element such that the detent contacts and rides over one or more of the protrusions during the rotation. Thus, the valve can operate more effectively as the outlet element is more suited to comprising two or more protrusions than the valve base. This is due to the rotation of the outlet element which allows each protrusion to move into and out of contact with the valve base as required.

The outlet element is rotatable such that the outlet tube moves through an arc of rotation and wherein at a first end of the arc of rotation, the outlet element is in the closed configuration, and at a second end of the arc of rotation, the outlet element is

in the open configuration. The detent is configured to engage at least one protrusion at a point during rotation of the outlet element that does not correspond to the first end of the arc of rotation. Accordingly in one embodiment of the invention, there is provided an outlet valve for an ostomy bag comprising: a valve base for attachment to a face of the ostomy appliance, the valve base defining a base opening for receiving a liquid output from the ostomy bag; and an outlet element configured to be rotatable relative to the valve base from a closed configuration in which discharge of the liquid received through the base opening is prevented, to an open configuration in which discharge of the liquid received through the base opening is enabled; wherein one of the outlet element and the valve base comprises a detent and the other of the outlet element and the valve base comprises one or more protrusions; and wherein the detent is configured to engage the one or more protrusions during a rotation of the outlet element such that the detent contacts and rides over one or more of the protrusions during the rotation, wherein the outlet element is rotatable such that the outlet tube moves through an arc of rotation, and wherein at a first end of the arc of rotation, the outlet element is in the closed configuration, and at a second end of the arc of rotation, the outlet element is in the open configuration, and wherein the detent is configured to engage at least one protrusion at a point during rotation of the outlet element that does not correspond to the first end of the arc of rotation. Thus, the detent/protrusions engage at a point not corresponding to the first end of the arc of rotation, allowing the user better control/feedback as the outlet element is moved from the closed to open positions and vice versa.

The detent may be configured to engage at least one protrusion at a point during rotation of the outlet element that corresponds to the second end of the arc of rotation. Thus, the outlet element is securely retained in the closed configuration.

The detent may be configured to engage at least one protrusion when the outlet element is at, or near a limit position. The limit position may be defined as a point on the arc of rotation at which the outlet element is furthest from the first end and also still in the closed configuration. Thus, the user can easily judge when the valve is about to open and therefore control the valve more efficiently and effectively.

As described above, the detent/protrusions in the present invention are arranged to improve the usability of the valve. Accordingly in a preferred embodiment of the invention, there is provided an outlet valve for an ostomy bag comprising: a valve base for attachment to a face of the ostomy appliance, the valve base defining a base opening for receiving a liquid output from the ostomy bag; and an outlet element configured to be rotatable relative to the valve base from a closed configuration in which discharge of the liquid received through the base opening is prevented, to an open configuration in which discharge of the liquid received through the base opening is enabled; wherein one of the outlet element and the valve base comprises a detent and the other of the outlet element and the valve base comprises one or more protrusions; and wherein the detent is configured to engage the one or more protrusions during a rotation of the outlet element such that the detent contacts and rides over one or more of the protrusions during the rotation, wherein the valve base comprises the detent, and the outlet element comprises two or more protrusions, and wherein the outlet element is rotatable such that the outlet tube moves through an arc of rotation, and wherein at a first end of the arc of rotation, the outlet element is in the closed configuration, and at a second end of the arc of rotation, the outlet element is in the open configuration, and wherein the detent is configured to engage at least one protrusion at a point during rotation of the outlet element that corresponds to the first end of the arc of rotation, and wherein the detent is configured to engage at least one protrusion when the outlet element is at, or near a limit position, wherein the limit position is defined as a point on the arc of rotation at which the outlet element is furthest from the first end and also still in the closed configuration.

The outlet element comprises an outlet tube at its distal end. The outlet tube may be rotatable in a plane generally perpendicular to an attachment face of the valve base. The outlet element is rotatable such that the outlet tube moves through an arc of rotation;
wherein at a first end of the arc of rotation, the outlet element is in the closed configuration, and at a second end of the arc of rotation, the outlet element is in the open configuration;
wherein a limit position of the outlet tube is defined at a point on the arc of rotation furthest from the first end at which the outlet element is still in the closed configuration; and
wherein a protrusion of the one or more protrusions is arranged such that the protrusion contacts the detent when the outlet element is at or near the limit position during rotation.

The outlet element may be configured such that in the limit position, in use, the outlet tube is angled generally upwards and at an acute angle to the valve base.

The outlet element outlet element may define an intake opening in fluid connection with the outlet tube, wherein:
in the open configuration the base opening and the intake opening are partially or fully aligned such that liquid may be received into the intake opening from the base opening and discharged through the outlet tube; and
in the closed configuration the base opening and the intake opening are not aligned such that discharge of the liquid received through the base opening is prevented. The intake opening may be arranged in a curved lateral wall of the outlet element.

The outlet element may further comprise an intake tube, the intake tube and the outlet tube together defining a continuous hollow bore for passage of the liquid from the intake opening to an outlet opening arranged at an end of the outlet tube distal to the intake tube.

The outlet element may have a generally L-shaped form, the intake tube optionally being arranged at an angle of substantially 90 degrees to the outlet tube. The housing may protrude from an outer face of the valve base, the housing defining a cylindrical bore, a portion of the intake tube being rotatably mounted with the cylindrical bore. The detent may be arranged on the outer face of the valve base adjacent to the housing and the one or more protrusions are arranged on the intake tube adjacent to the housing.

Additionally, or alternatively, in some embodiments the outlet element may rotate about an axis of rotation arranged generally parallel to an attachment face of the valve base and generally horizontally in use.

Additionally, or alternatively, in some embodiments the detent may be arranged on the valve base and the one or more protrusions are arranged on the outlet element.

Additionally, or alternatively, in some embodiments in the closed configuration the base opening may be obstructed by a wall of the outlet element.

Additionally, or alternatively, in some embodiments the detent may formed of a material which is less rigid than a material forming the one or more protrusions.

Additionally, or alternatively, in some embodiments the detent may be integrally formed with the valve base, and optionally the valve base and the detent may be formed as a single moulding.

Additionally, or alternatively, in some embodiments the one or more protrusions may be integrally formed with the outlet element, and optionally the outlet element and the one or more protrusions may be formed as a single moulding.

The present disclosure also provides an ostomy appliance comprising an outlet valve as described above and an ostomy bag, wherein the outlet valve is attached to an outer face of the ostomy bag.

Additionally, or alternatively, in some embodiments in the closed configuration the outlet element may substantially overlie a cavity of the ostomy bag; and in the open configuration at least a portion of the outlet element may extend across a lower edge of the cavity of the ostomy bag.

The present disclosure also provides an ostomy appliance comprising:
a valve base for attachment to a face of the ostomy appliance, the valve base defining a base opening for receiving a liquid output from the ostomy appliance; and
an outlet element configured to be rotatable relative to the valve base from a closed configuration in which discharge of the liquid received through the base opening is prevented, to an open configuration in which discharge of the liquid received through the base opening is enabled;
wherein the outlet element comprises a relatively rigid component rotatably attached to the valve base and a relatively flexible outlet tube portion affixed to the relatively rigid component.

In the open configuration the relatively rigid component may define a fluid pathway between the base opening and the relatively flexible outlet tube. Accordingly in one embodiment, there is provided an outlet valve for an ostomy appliance comprising: a valve base for attachment to a face of the ostomy appliance, the valve base defining a base opening for receiving a liquid output from the ostomy appliance; and an outlet element configured to be rotatable relative to the valve base from a closed configuration in which discharge of the liquid received through the base opening is prevented, to an open configuration in which discharge of the liquid received through the base opening is enabled; wherein the outlet element comprises a relatively rigid component rotatably attached to the valve base and a relatively flexible outlet tube portion affixed to the relatively rigid component, wherein in the open configuration the relatively rigid component is arranged to define a fluid pathway between the base opening and the relatively flexible outlet tube. Thus, the relatively rigid component carries the fluid to the relatively flexible outlet tube portion. This can allow the valve to be easily operated by the user (by rotating the relatively rigid component) and can avoid the part of the outlet element that fits into the valve base deforming during operation of the valve.

In the open configuration the relatively rigid component may be configured to receive liquid directly from the base opening and then deliver it to the relatively flexible outlet tube portion. Accordingly in one embodiment, there is provided an outlet valve for an ostomy appliance comprising: a valve base for attachment to a face of the ostomy appliance, the valve base defining a base opening for receiving a liquid output from the ostomy appliance; and an outlet element configured to be rotatable relative to the valve base from a closed configuration in which discharge of the liquid received through the base opening is prevented, to an open configuration in which discharge of the liquid received through the base opening is enabled; wherein the outlet element comprises a relatively rigid component rotatably attached to the valve base and a relatively flexible outlet tube portion affixed to the relatively rigid component, wherein in the open configuration the relatively rigid component is configured to receive liquid directly from the base opening and then deliver it to the relatively flexible outlet tube. Thus, fluid passes through the base opening and into the relatively rigid component; this means that the relatively rigid component controls the opening/closing of the valve, and ensures the outlet element can be rotated easily. The relatively rigid component then delivers the fluid to the relatively flexible outlet tube portion. Thus the flexible component can be provided further from the valve base which can make the valve easier to use.

In some embodiments, the relatively rigid component may have a tubular form. It may have a generally L-shaped form. Accordingly in one embodiment, there is provided an outlet valve for an ostomy appliance comprising: a valve base for attachment to a face of the ostomy appliance, the valve base defining a base opening for receiving a liquid output from the ostomy appliance; and an outlet element configured to be rotatable relative to the valve base from a closed configuration in which discharge of the liquid received through the base opening is prevented, to an open configuration in which discharge of the liquid received through the base opening is enabled; wherein the outlet element comprises a relatively rigid component rotatably attached to the valve base and a relatively flexible outlet tube portion affixed to the relatively rigid component, wherein the relatively rigid component has a tubular and generally L-shaped form. Thus, the relatively rigid component can transmit the fluid to the relatively flexible outlet tube portion effectively; can have a shape that is easily grasped by the user to operate the valve, and presents the relatively flexible outlet tube in a convenient position for connecting to other apparatus.

In some embodiments the relatively flexible outlet tube portion may be formed from a deformable material, wherein the deformable material is optionally resilient.

Additionally, or alternatively, in some embodiments the relatively rigid component may be formed from a material having a Shore hardness of at least 50A.

Additionally, or alternatively, in some embodiments the outlet element may comprise an outlet tube, the outlet tube comprising the relatively flexible outlet tube portion.5. The relatively rigid component may comprise an intake tube and a relatively rigid outlet tube portion, the relatively rigid outlet tube portion being affixed to the relatively flexible outlet tube portion. The intake tube and the outlet tube may together define a continuous hollow bore for passage of the liquid from the intake opening to an outlet opening arranged at an end of the outlet tube distal to the intake tube. The outlet element may have a generally L-shaped form, the intake tube optionally being arranged at an angle of substantially 90 degrees to the outlet tube. A housing may protrude from an outer face of the valve base, the housing defining a cylindrical bore, a portion of the intake tube being rotatably mounted with the cylindrical bore.

Additionally, or alternatively, in some embodiments the outlet element may be rotatable about an axis of rotation arranged generally parallel to an attachment face of the valve base and generally horizontally in use.

Additionally, or alternatively, in some embodiments the outlet valve may be configured for use with a relatively rigid connector having a connector diameter, the relatively flexible outlet tube portion having an outlet diameter marginally smaller than the connector diameter such that, on insertion of the relatively rigid connector into the relatively flexible outlet tube portion in use, the relatively flexible outlet tube portion flexes to accommodate the relatively rigid connector.

Additionally, or alternatively, in some embodiments the relatively flexible outlet tube portion may be over-moulded onto the relatively rigid component. Accordingly in one embodiment, there is provided an outlet valve for an ostomy appliance comprising: a valve base for attachment to a face of the ostomy appliance, the valve base defining a base opening for receiving a liquid output from the ostomy appliance; and an outlet element configured to be rotatable relative to the valve base from a closed configuration in which discharge of the liquid received through the base opening is prevented, to an open configuration in which discharge of the liquid received through the base opening is enabled; wherein the outlet element comprises a relatively rigid component rotatably attached to the valve base and a relatively flexible outlet tube portion affixed to the relatively rigid component, wherein the relatively flexible outlet tube portion is over-moulded onto the relatively rigid component.

Additionally, or alternatively, in some embodiments the valve base may be formed of a material which is more rigid than the relatively flexible outlet tube portion and optionally which is less rigid than the relatively rigid component.

Additionally, or alternatively, in some embodiments the relatively rigid component may comprise a protruding flange for gripping by the user in use. Accordingly, in one embodiment, there is provided an outlet valve for an ostomy appliance comprising: a valve base for attachment to a face of the ostomy appliance, the valve base defining a base opening for receiving a liquid output from the ostomy appliance; and an outlet element configured to be rotatable relative to the valve base from a closed configuration in which discharge of the liquid received through the base opening is prevented, to an open configuration in which discharge of the liquid received through the base opening is enabled; wherein the outlet element comprises a relatively rigid component rotatably attached to the valve base and a relatively flexible outlet tube portion affixed to the relatively rigid component, wherein the relatively rigid component comprises a protruding flange for gripping by the user in use.

The present disclosure also provides an ostomy appliance comprising an outlet valve as as described above. In the closed configuration the outlet element may substantially overlie a cavity of the ostomy appliance; and in the open configuration at least a portion of the outlet element may extend across a lower edge of the cavity of the ostomy appliance.

### Brief description of the drawings

One or more aspects of the disclosure will now be described, by way of example only, with reference to the accompanying drawings in which:
Figure 1 illustrates a schematic perspective view of an outlet valve for an ostomy appliance according to the invention with the outlet element in the closed configuration at a first end of its arc of rotation;
Figure 2 illustrates a schematic perspective view of the outlet valve of Figure 1 with the outlet element in the open configuration at a second end of its arc of rotation;
Figure 3 illustrates a schematic perspective view of an outlet element of the outlet valve of Figure 1;
Figure 4 illustrates a further schematic perspective view of the outlet element of Figure 3;
Figure 5 illustrates a schematic perspective view of a valve base of the outlet valve of Figure 1;
Figure 6 illustrates a further schematic perspective view of the valve base of Figure 5;
Figure 7 illustrates a schematic perspective view of an outlet valve for an ostomy appliance not according to the invention and present for illustration purposes only with the outlet element in closed configuration at a first end of its arc of rotation;
Figure 8 illustrates a schematic perspective view of the outlet valve of Figure 7 with the outlet element in the open configuration at a second end of its arc of rotation; and
Figure 9 illustrates a schematic perspective view of an outlet valve for an ostomy appliance not according to the invention and present for illustration purposes only.

### Detailed description

In the following description, the equivalent reference numerals are used in different aspects to denote equivalent or similar features.

Unless defined otherwise, all technical and scientific terms used in this specification have the same meaning as is commonly understood by the reader skilled in the art to which the claimed subject matter belongs. It is to be understood that the foregoing summary of the disclosure and the following examples are exemplary and explanatory only and are not restrictive of any subject matter claimed.

The following description is directed to exemplar aspects of the disclosure. The description of the aspects is not meant to include all the possible aspects of the disclosure that are claimed in the appended claims. Many modifications, improvements and equivalents which are not explicitly recited in the following aspects may fall within the scope of the appended claims. Features described as part of one aspect may be combined with features of one or more other aspects unless the context clearly requires otherwise.

In this specification, the use of the singular includes the plural unless the context clearly dictates otherwise. In this specification, the use of "or" means "and/or" unless stated otherwise. Furthermore, use of the term "including" as well as other forms, such as "include", "includes," and "included," is not limiting.

As used herein, ranges and amounts can be expressed as "about" a particular value or range. About also includes the exact amount. For example, "about 5 mm" means "about 5 mm" and also "5 mm." Generally, the term "about" includes an amount that would be expected to be within experimental error. The term "about" includes values that are within 10% less to 10% greater of the value provided. For example, "about 50%" means "between 45% and 55%." Also, by way of example, "about 30" means "between 27 and 33."

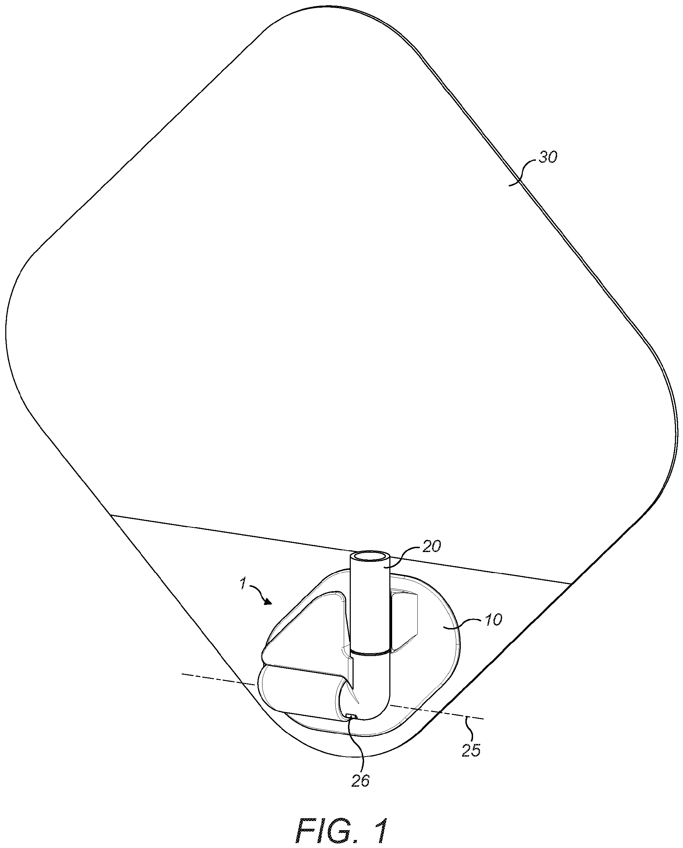

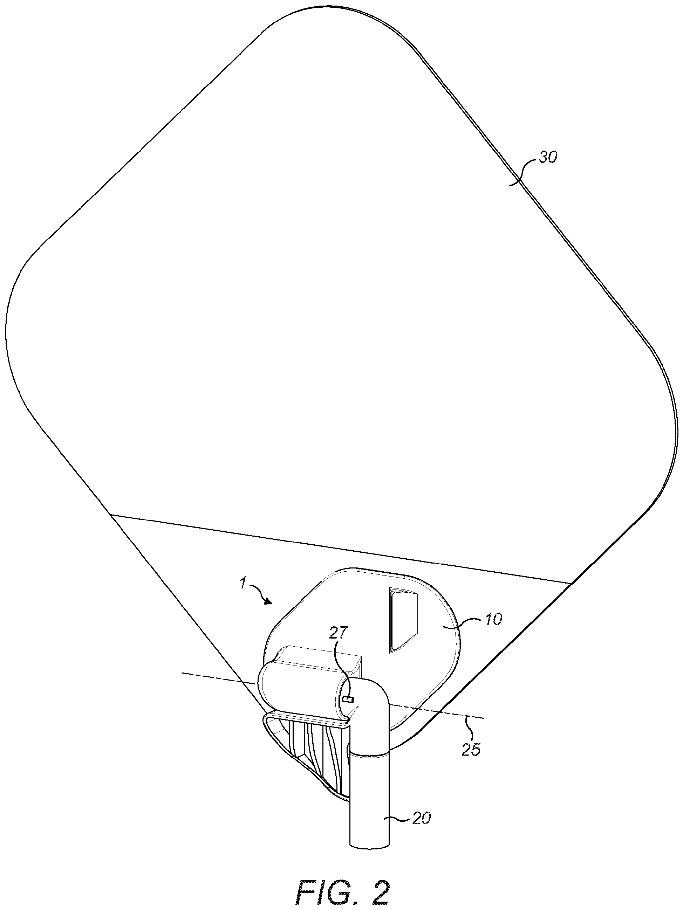

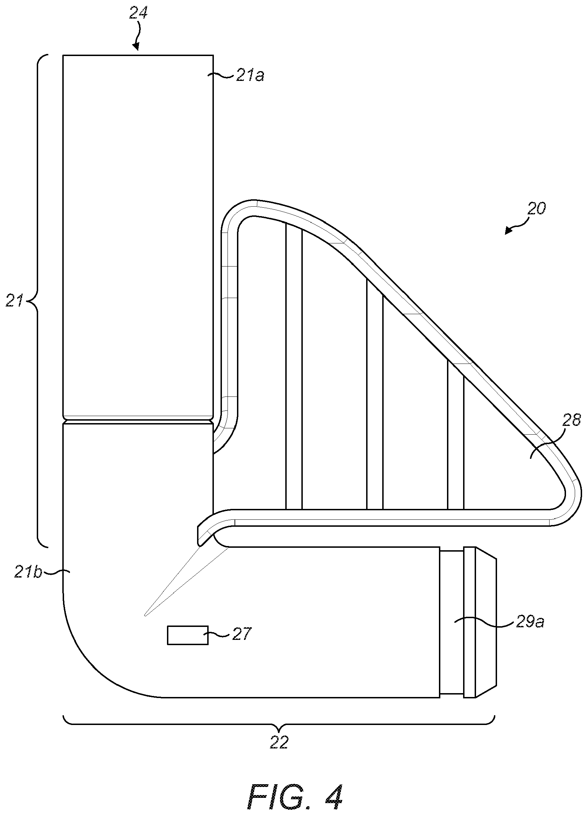

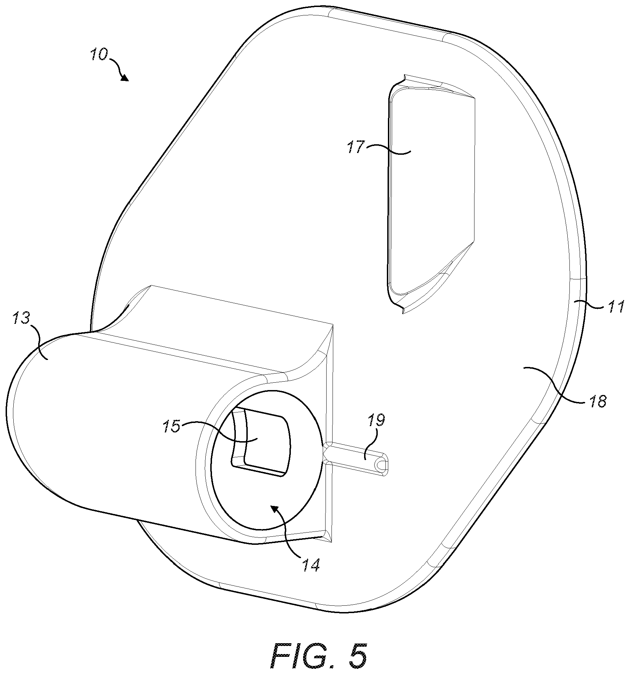

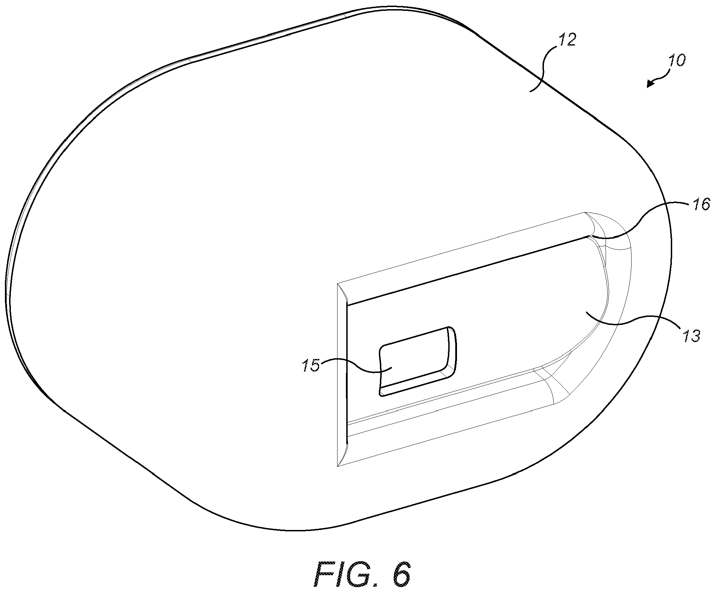

An outlet valve 1 for an ostomy appliance according to the present invention is shown in Figures 1 to 6. The ostomy appliance may comprise an ostomy bag (shown in Figures 1 and 2) and the outlet valve 1, the outlet valve 1 being attached to an outer face of an ostomy bag 30, preferably to an outer face of an outer wall of the ostomy bag 30.

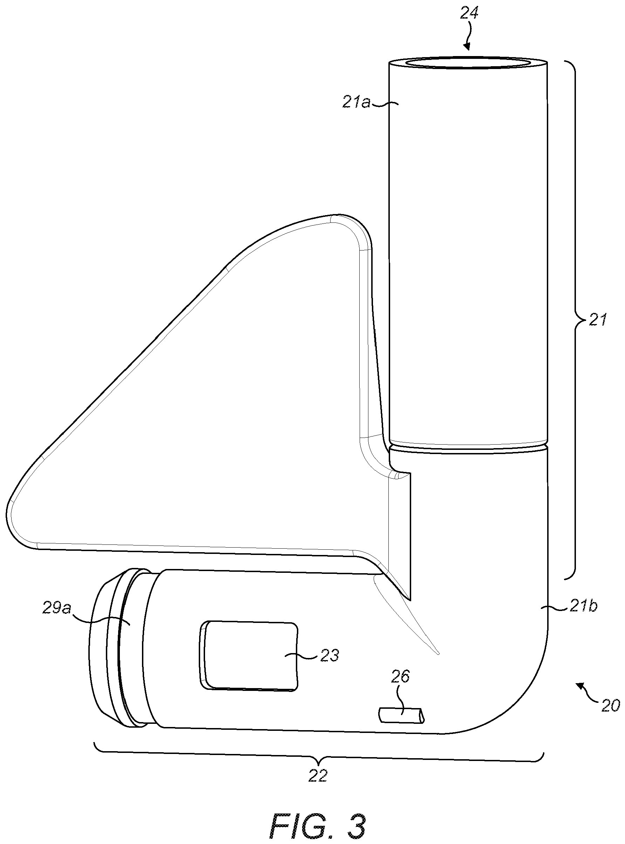

The outlet valve comprises a valve base 10 and an outlet element 20. The valve base 10 and outlet element 20 are shown assembled in Figures 1 and 2, and disassembled in Figures 3 to 6. The disassembled components are shown for clarity only. The outlet valve 1 is not configured to be disassembled in normal use.

The valve base 10, as shown in Figures 1, 2, 5 and 6, is configured for attachment to the outer face of the ostomy bag 30 and may comprise a backing flange 11 having a generally planar attachment face 12 arranged on a back side of the valve base 10 (i.e. a face configured to be proximal to the body of the ostomate in use). The generally planar attachment face 12 may be attachable to the outer face of the ostomy bag 30, for example by welding or by using an adhesive.

A housing 13 of the valve base 10 may protrude from an outer face 18 of the back flange 11. The housing 13 may be integrally formed with the backing flange 11, for example by moulding. The housing 13 may define a generally cylindrical bore 14 for rotatable attachment to the outlet element 20 in use.

A base opening 15 is provided through the valve base 10 for receiving a liquid output from the ostomy bag 30. An ostomy bag aperture may be provided in a wall of the ostomy bag 30 through which liquid may be output from the ostomy bag 30 to the base opening 15, the outlet valve 1 being arranged overlying the ostomy bag aperture. The base opening 15 may be arranged within the cylindrical bore 14 of the housing 13 such that base opening 15 may be in liquid communication with a cavity of the ostomy bag 30. The base opening 15 may have an elongate shape, preferably generally parallel in its elongate direction to an axis of rotation 25 of the outlet element 20. The base opening 15 may have a generally rectangular shape, preferably with rounded corners.

A recess 16 may be formed in the generally planar attachment face 12 around the base opening 15. The presence of the recess 16 may allow for the housing 13 to have a generally constant wall thickness while providing the other required features, thereby facilitating moulding of the housing 13.

A ridge 17 for abutting a portion of the outlet element 20 when it in the closed configuration is provided on the outer face 18 of the backing flange 11. The ridge 17 may provide a barrier to restrict access to at least a part of the outlet element 20 when it is in the closed configuration, thereby avoiding unintended rotation of the outlet element 20. The ridge 17 may also provide an indication that the nozzle is in the closed configuration.

The outlet element 20 is configured to be rotatable relative to the valve base 10 from a closed configuration in which discharge of the liquid received through the base opening 15 is prevented, to an open configuration in which discharge of the liquid received through the base opening 15 is enabled.

The outlet element 20 may be rotatably mounted to the housing 14, and may define an intake opening 23 configured to align with and receive liquid from the base opening 15 in use. The outlet element 20, as shown in Figures 1 to 4, may comprise an outlet tube 21 for discharging fluid from the outlet valve 1 to the exterior or to a further appliance to be attached to the outlet tube 20 in use. The outlet tube 21 may be fluidly connected to an intake tube 22. The intake tube 22 and the outlet tube 21 together may define a continuous hollow bore for passage of the liquid from the intake opening 23 to an outlet opening 24 arranged at an end of the outlet tube 21 distal to the intake tube 22.

The outlet element 20 may comprise a relatively rigid component rotatably attached to the valve base 10 and a relatively flexible outlet tube portion 21a affixed to the relatively rigid component. The relatively flexible outlet tube portion 21a may be formed from a resiliently deformable material. The relatively flexible outlet tube portion 21a may form a part of the outlet tube 21 distal to the intake tube 22 and may comprise the outlet opening 24.

The relatively rigid component may be formed from a material having a Shore hardness of at least about 50A. The relatively rigid component may comprise the intake tube 22 and a relatively rigid outlet tube portion 21b, the relatively flexible outlet tube portion 21a being affixed to the relatively rigid outlet tube portion 21b, for example by over-moulding the relatively flexible outlet tube portion 21a onto the relatively rigid outlet tube portion 21b.

A portion of the intake tube 22 may be rotatably mounted with the cylindrical bore 14 of the housing 15. The outlet element 20 may therefore rotate about an axis of rotation 25, being the longitudinal axis of the cylindrical bore 14. In use, the outlet valve 1 may be arranged on the ostomy bag 30 such that in a closed configuration the outlet tube 21 may be arranged with the outlet opening 24 directed upwards, and in the open configuration the outlet tube 21 may be arranged with the outlet opening 24 directed downwards.

An open end of the intake tube 22 may be fluidly sealed by contact with an adjacent wall of the housing 13.

The intake tube 21 may be retained in the housing 13 by a retention feature 29 comprising an annular recess 29a arranged on the intake tube 22 and a corresponding annular protrusion arranged on a wall of the housing inside the cylindrical bore 14. The retention feature 29 may form a seal between the cylindrical bore 14 and the outer surface of the intake tube 22. The retention feature 29 may prevent axial movement of the outlet element 20 relative to the valve base 10 during rotation.

The axis of rotation 25 may be arranged generally parallel to the attachment face 12 of the valve base 10 and may be generally horizontal in use. The outlet element 20 may have a L-shaped form, the intake tube 22 being arranged at an angle of substantially 90 degrees to the outlet tube 21. The outlet tube 21 may therefore be rotatable in a plane generally perpendicular to the attachment face 12 of the valve base 10 and generally vertical in use.

The outlet element 20 may be rotatable such that the outlet tube 21 moves through an arc of rotation from an upward-pointing position shown in Figure 1 (i.e. with the outlet opening 24 uppermost) at a first end of the arc of rotation to a downward-pointing position at a secondend of the arc of rotation as shown in Figure 2.

The intake opening 23 may be arranged in the intake tube 22 such that the intake opening 23 is in fluid connection the outlet opening 24 via the hollow bore arranged through the intake tube 22 and the outlet tube 21. As shown in Figure 3, the intake opening 23 may be arranged in a portion of a curved lateral wall of the intake tube 22 received in the housing 13 such that the inlet opening 23 may be rotated into and out of alignment with the base opening 15 in use. The intake opening 23 may have an elongate shape, preferably generally parallel in its elongate direction to an axis of rotation 25 of the outlet element 20. The intake opening 23 may have a generally rectangular shape, preferably with rounded corners. The intake opening 23 may have a shape matching that of the base opening 15.

In the open configuration of the outlet element 20, the base opening 15 and the intake opening 23 are partially or fully aligned such that liquid may be received into the intake opening 23 from the base opening 15 and discharged through the outlet tube 21. In the closed configuration the base opening 15 and the intake opening 23 are not aligned such that discharge of the liquid received through the base opening 15 is prevented. In the closed configuration passage of liquid from the base opening 15 into the intake tube 22 may be obstructed by an adjacent portion of a wall of the intake tube 23.

The base opening 15 and the intake opening 23 may be positioned such that when the outlet element 20 is arranged at the first end of the arc of rotation, the outlet element 20 is in the closed configuration. In use the outlet element 20 may be rotated downwards away from the first end of the arc of rotation. The intake opening 23 may move closer to the base opening 15 as the outlet element is rotated. A limit position of the outlet tube 21 may be defined at a point on the arc of rotation furthest from its first end at which the outlet element 20 is still in the closed configuration. On further rotation of the outlet element 20 away from the first end and past the limit position, the base opening 15 may start to come into alignment with, and then progressively align with, the intake opening 23 such that liquid may pass from the base opening 15 into the intake opening 23 and the outlet element 20 is therefore in the open configuration. Continued rotation of the outlet element 20 away from the first end increases the alignment of the base opening 15 and the intake opening 23 such that the resulting flow path through the base and intake openings increases in cross section. At a second end of the arc of rotation, the outlet element 20 may be in the open configuration with the base opening 15 and the intake opening 23 fully aligned.

The outlet valve 1 may be configured such that in the limit position in use, the outlet tube 21 is angled generally upwards and at an acute angle to the attachment face 12 of the valve base 10.

The outlet element 20 may comprise a first protrusion 27 and a second protrusion 26 arranged around the circumference of an outer face of the intake tube 22, adjacent to the portion of the intake tube 22 received in the cylindrical bore 14 of the housing 13. The first and second protrusions 26, 27 may be integrally formed with the intake tube 22, for example being formed in a single moulding with the intake tube 22. The first protrusion 27 and the second protrusion 26 may be arranged at the same distance along the intake tube 22 and spaced apart around the circumference of the intake tube 22.

The valve base 10 may comprise a detent 19 in the form of an elongate protrusion arranged on the outer face 18 of the back flange 11, outside and adjacent to the housing 13. The detent 19 may be adjacent to an opening of the cylindrical bore 13. The detent 19 may be aligned with the longitudinal axis of the cylindrical bore 14 and therefore the axis of rotation 25 of the outlet element 20.

The detent 19 and the first protrusion 27 may be positioned such that on rotation of the outlet tube 21 away from the first end of its arc of rotation, the first protrusion 26 contacts and rides over the detent 19, thereby providing audible and/or tactical confirmation to the user that the outlet tube 21 has been moved out of its initial storage position.

The detent 19 and the second protrusion 26 may be positioned such that on rotation of the outlet element to or near the limit position, the first protrusion 26 contacts and rides over the detent 19, thereby providing audible and/or tactical confirmation to the user that the outlet tube 21 has reached or is approaching the limit position.

The backing flange 11 may have a length from the axis of rotation 25 to its upper edge which is shorter than the length of the outlet tube 20 from the axis of rotation to the outlet opening 24. The outlet opening 24 may therefore be arranged outside a footprint of the backing flange 11 (i.e. does not overlie the backing flange 11) when the outlet element 20 is in the closed configuration at the first, upper end of its arc of rotation, as shown in Figure 1.

The relatively rigid outlet tube portion 21b may have a length from its axis of rotation 25 to the relatively flexible outlet tube portion 21a which is shorter than a length of the backing flange 11 from a line parallel to the axis of rotation 25 to a lower edge of the backing flange 11such that at the second, lower end of its arc of rotation the distal end of the relatively rigid outlet tube portion 21b (distal to the intake tube 22) overlies the backing flange 11. The relatively flexible outlet tube portion 121a may extend from the distal end of the relatively rigid outlet tube portion 21b. Therefore, a portion of the relatively flexible outlet tube portion 21a may be arranged across the lower edge of the backing flange 11 such that a distal portion of the relatively flexible outlet tube portion 21a may be arranged outside a footprint of the backing flange 11 when the outlet element 20 is at the second, lower end of its arc of rotation. The outlet valve 1 may be arranged adjacent to a lower edge or lower apex of the ostomy bag 30 such that all of the relatively flexible outlet tube portion 21a may be arranged outside a footprint of the ostomy bag 30 when the outlet element 20 is at the second, lower end of its arc of rotation.

In use, the outlet valve 1 may be attached to an outer face of an ostomy bag 30 (shown in Figures 1 and 2). The outlet valve 1 may be attached adjacent to a lower edge or lower apex of the ostomy bag 30. In the closed configuration, the outlet element 20 may substantially overlie a cavity of the ostomy bag 30. In the open configuration, at the second end of its arc of rotation, at least a portion of the outlet element 20 may extend across a lower edge of the cavity of the ostomy bag 30. As shown in Figure 2, the relatively flexible outlet tube and/or a relatively rigid portion of the outlet tube may extend across the lower edge of the cavity such that the outlet opening 24 does not overlie the cavity of the ostomy bag 30 when the outlet element 20 is in the open configuration at the second end of its arc of rotation.

A grip flange 28 may be attached to the outlet element 20 for gripping by the user to rotate the outlet element 20 between the open and closed configurations. The flange 28 may extend laterally from the outlet tube 21. The flange 28 may be integrally formed with the rigid portion of the outlet element 20. The flange 28 may have a generally triangular form and may be provided with surface features to assist in gripping the flange by the user. The backing flange 11 may comprise a lateral flange extending laterally on an opposite side of the outlet valve to the grip flange 28, thereby providing a gripping surface for the user to press or hold while moving the outlet element 20 using the grip flange 28.

Further examples of an outlet valve according to the present disclosure are described below. Only those features that differ in this aspect compared to the previous aspect will be described in detail in the following description. For features that are common to one or more aspects, reference should be made to the description as a whole.

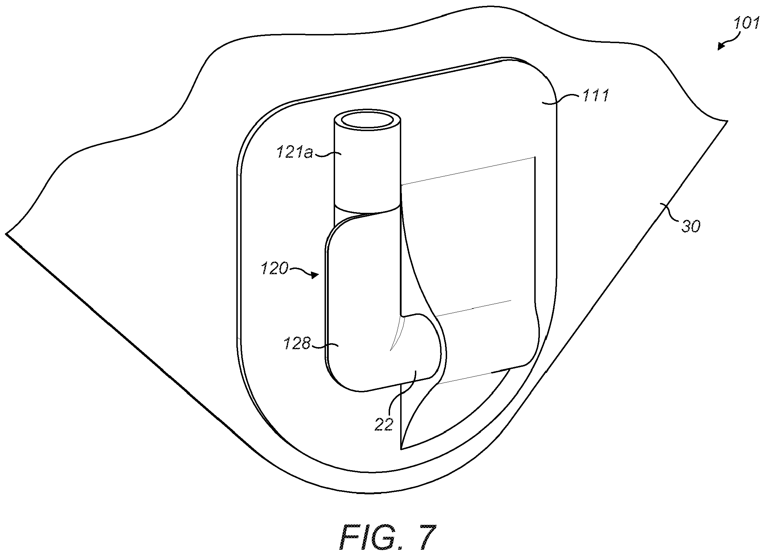

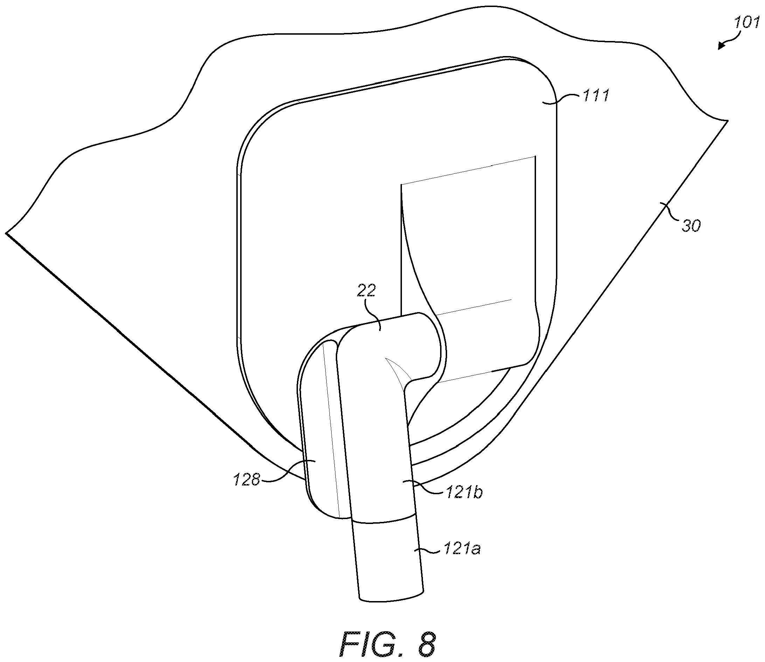

An outlet valve 101 for an ostomy appliance not according to the present invention and present for illustration purposes only is shown in Figures 7 and 8.

The backing flange 111 may have a length from a line parallel to the axis of rotation 25 to its upper edge which is longer than the length of the outlet tube 120 from the axis of rotation to the outlet opening 24. The outlet opening 24 may therefore be arranged inside a footprint of the backing flange 111 (i.e. overlies the backing flange 111) when the outlet element 120 is at the first end of its arc of rotation, as shown in Figure 8.

The relatively rigid outlet tube portion 121b may have a length from its axis of rotation 25 to the relatively flexible outlet tube portion 121a which is the same as or longer than a length of the backing flange 111 from a line parallel to the axis of rotation 25 to its lower edge such that at the second, lower end of its arc of rotation the distal end of the relatively rigid outlet tube portion 121b (distal to the intake tube 22) is arranged beyond a lower edge of the backing flange 111. Therefore, all of the relatively flexible outlet tube portion 121a may be arranged outside a footprint of the backing flange 111 when the outlet element 120 is at the second, lower end of its arc of rotation. The outlet valve 101 may be arranged adjacent to a lower edge or lower apex of the ostomy bag 30 such that all of the relatively flexible outlet tube portion 121a may be arranged outside a footprint of the ostomy bag 30 when the outlet element 120 is at the second, lower end of its arc of rotation.

The grip flange 128 may have a generally rectangular form. The generally rectangular grip flange 128 may have rounded corners.

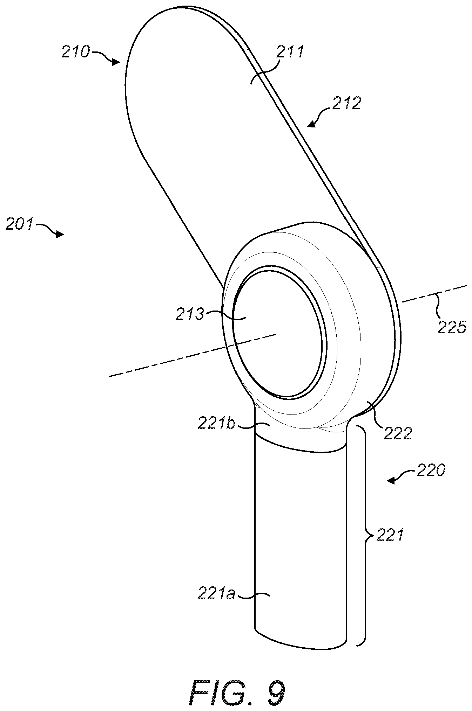

An outlet valve 201 for an ostomy appliance not according to the present invention and present for illustration purposes only is shown in Figure 9 in the open configuration at the second, lower end of its arc of rotation.

The valve base 210 of outlet valve 201 may comprise a backing flange 211 and a housing in the form of an axle 213. The axle 213 comprises a hollow bore for receiving liquid from the ostomy bag 30 and a base opening arranged in a wall of the axle 213.

The outlet valve 201 may be configured such that in use the axis of rotation 225 the axis of rotation is arranged generally horizontally but perpendicular to an attachment face 212 of the backing flange 211 of the valve base 210.

The outlet element 220 of outlet valve 201 may comprise an outlet tube 221 and an intake portion in the form of a cap 222. The cap 222 may be arranged concentrically with the axle 213. The cap 222 may be rotatable around the axle 213 to move the outlet element 220 from the closed configuration to the open configuration. The cap 222 may comprise an intake opening which may be brought into alignment with the base opening by rotation of the outlet element 220 such that a flow path is opened between from the base opening to the outlet tube 221.

One of the outlet element 220 and the valve base 210 may comprise a detent and the other of the outlet element 220 and the valve base 210 may comprise one or more protrusions. The detent may be configured to engage the one or more protrusions during a rotation of the outlet element such that the detent contacts and rides over one or more of the protrusions during the rotation. The detent may be arranged on the cap 222. The one or more protrusions may be arranged on the axle 213 or on the backing flange 211 of the valve base 210.

The outlet element 220 may comprise relatively flexible outlet tube portion. The relatively flexible outlet tube portion 221a and relatively rigid outlet tube portion 221b may be as described in the above aspects of the present disclosure.

In use, to drain liquid from an ostomy appliance comprising an outlet valve according to the present disclosure, the user may grip the grip flange and rotate the outlet element away from the first, upward end of its arc of rotation. A first protrusion and the detent may be positioned such that on movement of the bag audible and/or tactile feedback is provided to the user to confirm movement of the outlet element out of the upward position. At or near the limit position, a second protrusion may contact the detent, indicating the limit point in the arc of rotation beyond which the base opening and the inlet opening begin to align to open a fluid path from the base opening into the outlet tube. At the limit position, the outlet tube may be angled upwards and away from the user's body, thereby providing a convenient position for attachment of a drain appliance such as a night bag to the outlet tube before the outlet tube is moved into the open configuration, thereby limiting the possibility of spillage of the output liquid. The outlet tube may then be rotated further, the second protrusion passing the detent, to increase the alignment of the base and intake openings and thereby allow more fluid to flow out of the valve, and to orient the outlet tube downwards for convenient drainage into the night bag or to the exterior.

After drainage of the ostomy bag, or when the user wishes to interrupt the flow of liquid out of the outlet valve, the user may rotate the outlet tube upwards. On reaching or passing the limit position, the second protrusion will come into contact with the detent, confirming to the user that the base opening and the intake opening are no longer aligned. If used, a night drainage bag or other appliance can be disconnected from the outlet tube once the outlet element is in the closed configuration, avoiding spillages. On continued rotation, the first protrusion and detent will provide audible and/or tactile feedback when the outlet element reaches the upper end of its arc of rotation, the outlet opening facing upwards, confirming to the user that he outlet valve is securely closed.

The outlet valve according to any aspect of the disclosure may be configured for use with a relatively rigid connector, for example for connecting the outlet valve to a drainage bag or other appliance. The relatively rigid connector may have an outer diameter and the relatively flexible outlet tube portion of the outlet valve may have an outlet diameter marginally smaller than the connector outer diameter such that, on insertion of the connector into the relatively flexible outlet tube portion in use, the relatively flexible outlet tube portion may flex to accommodate the connector and thereby form an effective seal.

Whilst preferred aspects of the present disclosure have been described, these are by way of example only and non-limiting. It will be appreciated by those skilled in the art that many alternatives are possible within the ambit of the disclosure. For example, features described as part of one aspect may be combined with features of one or more other aspects unless the context clearly requires otherwise. Further, the following features may be applicable to any aspect of the disclosure.

As noted above, in this specification locations and orientations of features may be described with reference to the ostomy appliance being "in use", "orientated as it would be in use" or similar. Such terms refer to the intended orientation of the ostomy appliance when it is adhered to a body of an ostomate with the ostomate in a standing position.

The axis of rotation of the outlet element may be arranged generally parallel to the attachment face of the valve base and may be generally horizontally in use, for example as in the aspects shown in Figures 1 to 8. The intake tube or cap may have a central longitudinal axis. The axis of rotation of the outlet element may therefore be the central rotational axis of the intake tube or cap.

The outlet element may have a L-shaped form, the intake tube being arranged at an angle of substantially 90 degrees to the outlet tube. The outlet tube may therefore be rotatable in a plane which is generally perpendicular to the attachment face of the valve base and generally vertical in use, about an axis perpendicular to a longitudinal axis of the outlet tube.

The intake opening and sealing surfaces surrounding it may therefore be arranged along and rotate around an axis parallel to the attachment face of the outlet valve. This may reduce the profile depth of the valve from back to front when the outlet element is in a storage position at the first end of its arc of rotation, when compared to an outlet valve in which the intake opening and sealing surfaces surrounding it are arranged along and rotate around an axis perpendicular to the attachment face of the outlet valve.

The intake tube may be fixedly attached or integrally formed with at least a portion of the outlet tube, such that the inlet tube is not rotatable from or rotatable relative to the outlet tube.

In use, the outlet opening may be oriented generally upwards when the outlet element is in the closed configuration at the first end of its arc of rotation and generally downwards when the outlet opening is in the open configuration at a second end of its arc of rotation.

At the first end of the arc of rotation, the outlet tube may lie in contact with the backing flange along substantially all of its length, such that the outlet valve does not protrude more than necessary away from the attachment face.

The outlet tube may rotate in a plane generally perpendicular to an attachment face of the valve base such that, in use, the plane of rotation of the outlet tube is generally parallel or at an acute angle to the sagittal plane of the user.

The outlet element may be configured such that in the limit position in use, the outlet tube is angled generally upwards and away from the body of the user (i.e. at an acute angle to the attachment face of the valve base).

In use, when the base opening and intake opening are at least partially aligned the outlet element is in the open configuration. As the outlet tube is moved from its limit position towards the second end of the arc of rotation the apertures will become gradually more aligned, increasing the area of the open portion of the aperture gradually until reaching a fully open configuration in which both apertures are aligned. The position at which the outlet element is in a fully open configuration, i.e. in which the base and intake openings are fully aligned, may coincide with the second end of the arc of rotation.

An ostomy bag of an ostomy appliance according to the present disclosure may comprise a weld or other sealing line arranged adjacent to and below an opening in the bag for passing liquid to the outlet valve, thereby limiting the amount of liquid collecting below the opening inside the ostomy bag.

The outlet element may comprise a relatively flexible outlet tube portion for attachment of the outlet tube to a drainage appliance such as a night bag. Alternatively, the outlet tube element may comprise a relatively rigid portion at its outlet end. Where present, the flexible outlet tube portion may be attached to a relatively rigid outlet tube portion by over-moulding the flexible component onto the rigid component.

The outlet tube may have a generally circular cross-section at the outlet end as shown in the first and second illustrated aspects. Alternatively, the outlet tube may comprise a different cross-sectional shape. For example, the outlet tube may be generally oval or generally flattened at the outlet tend. The cross section may have an elongate shape with two generally parallel longer sides, joined by shorter curved ends, the longer sides being arranged generally parallel to the attachment face of the valve base in use, thereby reducing the profile of the outlet valve.

The outlet element may be retained in or around the housing by one or more retention features. The retention feature(s) may comprise one or more annular recesses and corresponding annular protrusions. The retention feature(s) may comprise one or more detents and corresponding protrusions. The retention feature(s) may form a seal between the outlet element and the housing.

The relatively flexible outlet tube portion may have a resilient tubular form and may be permanently affixed to the relatively rigid portion of the outlet tube, defining a continuous bore for passage of liquid from the intake portion/opening to the outlet end.

The relatively flexible outlet tube portion may be attached to the relatively rigid outlet tube portion by over-moulding the flexible material onto the rigid material during manufacture, to thereby permanently affix the relatively flexible outlet tube portion. Alternatively, the relatively flexible outlet tube portion may be affixed by other means, for example using adhesives.

The one or more detents may be in the form of a further protrusion extending from a face of the component. In use, the one or more detents may contact and pass over one or more of the protrusions, thereby providing tactile and/or audible feedback to the user confirming the position of the outlet tube relative to the valve base.

In the illustrated aspects, a single detent and multiple protrusions are provided. In any aspect, different numbers of detents and/or protrusions may be provided.

In the illustrated aspects, the detent and protrusions are arranged outside the cylindrical bore of the housing. The detent may be arranged on an outer face of the valve base, outside and adjacent to the housing. Placing the detent(s) outside the cylindrical bore of the housing may prevent them affecting the fit of the intake tube in the cylindrical bore, may improve sealing. In any aspect, the detent(s) and or protrusion(s) may be provided in positions other than those shown in the illustrated aspects.

The valve base may be formed of a material which is relatively more rigid than the relatively flexible outlet tube portion and which is less rigid than the relatively rigid component of the outlet element. The detent(s) may be formed of a material which is relatively less rigid than a material forming the protrusion(s) to facilitate passing of the detent(s) and protrusion(s) over each other in use.

The valve base and relatively rigid component of the outlet element may be formed, for example, from polypropylene or High-density polyethylene (HDPE), acrylonitrile butadiene styrene (ABS). or polycarbonate. The materials of the valve base and relatively rigid component of the outlet element components may be chosen to have different shrinkage rates to allow for hot assembly of these two components to produce a good seal therefore preventing leakage. The use of different materials must also be different to avoid sticking between the two components over time.

The relatively flexible outlet tube portion may be formed, for example, from ethylene-vinyl acetate (EVA) or a thermoplastic elastomer (TPE) and may be over-moulded over the relatively rigid portion of the outlet tube.

The detent(s) may be integrally formed with the valve base. The valve base and the detent(s) may be formed as a single moulding. The one or more protrusions may be integrally formed with the outlet element. The outlet element and the one or more protrusions may be formed as a single moulding.

In any aspect, one or more stops may be provided for contacting the detent or protrusions or other the outlet element to prevent rotation of the outlet element beyond the ends of the arc of rotation.

The detent(s) may engage a protrusion when the outlet element rotates away from its storage position at the first end of its arc of rotation and on moving to or from the limit position. The protrusion arranged to indicate movement of the outlet element away from its storage position may also assist in retaining the outlet element in the storage position until deliberately rotated by the user. The protrusion arranged to indicate movement of the outlet element on moving to or from the limit position may be positioned to make contact with the detent when the outlet element is at the limit position or near to the limit position (such that the outlet element may remain in the closed configuration for a small amount of rotation after contacting the detent, such that the flow path is not opened immediately on moving the outlet element past this protrusion.

The detent(s) may be located on a section of the valve base that allows it to flex out of the way on contact with the protrusion(s) of the outlet element, such that the detent(s) may flex away rather than permanently deforming. This may reduce wear of the detent(s) and protrusion(s).

The intake tube may be assembled with the housing or axle using a hot assembly method, such that after shrinkage of the housing on cooling, the intake tube is retained within the housing.

The outlet element may comprise a grip flange as described above. The grip flange may be integrally formed with the relatively rigid component, or may be attached, for example by adhesive.

A lubricant may be provided between the rotating components to ease rotation and improve sealing.

In any aspect of the present disclosure, the outlet element may be retained in an axial direction relative to the housing, such that there is no relative axial movement between the outlet element and the valve base during rotation.

Other features of the ostomy appliance, for example the shape and construction of the ostomy appliance inner and outer walls and comfort layer, may vary from those shown in the illustrated aspects.

The outlet valve of the present disclosure is described herein as being suitable for an ostomy appliance comprising an ostomy bag. An outlet valve according to any aspect of the present disclosure may be suitable for use in other applications, for example a drainage bag or catheter collection bag.

## Claims

1. An outlet valve (1) for an ostomy bag (30) comprising: a valve base (10) for attachment to a face of the ostomy appliance, the valve base (10) defining a base opening (15) for receiving a liquid output from the ostomy bag (30); and an outlet element (20) configured to be rotatable relative to the valve base (10) from a closed configuration in which discharge of the liquid received through the base opening (15) is prevented, to an open configuration in which discharge of the liquid received through the base opening (15) is enabled; wherein one of the outlet element (20) and the valve base (10) comprises a detent (19) and the other of the outlet element (20) and the valve base (10) comprises one or more protrusions (26, 27); and wherein the detent (19) is configured to engage the one or more protrusions (26, 27) during a rotation of the outlet element (20) such that the detent (19) contacts and rides over one or more of the protrusions (26, 27) during the rotation, wherein the outlet element (20) comprises an outlet tube (21) at its distal end and the outlet element (20) is rotatable such that the outlet tube (21) moves through an arc of rotation, and wherein at a first end of the arc of rotation, the outlet element (20) is in the closed configuration, and at a second end of the arc of rotation, the outlet element (20) is in the open configuration, **characterised in that** the detent (19) is configured to engage at least one protrusion (26) at a point during rotation of the outlet element (20) that does not correspond to the first end of the arc of rotation.

2. An outlet valve (1) as claimed in claim 1 wherein one of the outlet element (20) and the valve base (10) comprises the detent (19) and the other of the outlet element (20) and the valve base (10) comprises two or more protrusions (26, 27).

3. An outlet valve (1) as claimed in claim 2 wherein the valve base (10) comprises the detent (19) and the outlet element (20) comprises the two or more protrusions (26, 27).

4. An outlet valve (1) as claimed in any preceding claim, wherein the detent (19) is further configured to engage at least one protrusion (26) at a point during rotation of the outlet element (20) that corresponds to the second end of the arc of rotation.

5. An outlet valve (1) as claimed in any preceding claim, wherein the detent (19) is configured to engage at least one protrusion (26) when the outlet element (20) is at, or near a limit position, wherein the limit position is defined as a point on the arc of rotation at which the outlet element (20) is furthest from the first end and also still in the closed configuration.

6. An outlet valve (1) as claimed in any preceding claim
wherein a limit position of the outlet tube (21) is defined at a point on the arc of rotation furthest from the first end at which the outlet element (20) is still in the closed configuration; and
wherein a protrusion (26) of the one or more protrusions (26, 27) is arranged such that the protrusion (26) contacts the detent (19) when the outlet element (20) is at or near the limit position during rotation.

7. An outlet valve (1) as claimed in claim 6 wherein the outlet element (20) is configured such that in the limit position, in use, the outlet tube (21) is angled generally upwards and at an acute angle to the valve base (10).

8. An outlet valve (1) as claimed in any one of claims 6 to 7 wherein the outlet element (20) defines an intake opening (23) in fluid connection with the outlet tube (21), wherein:
in the open configuration the base opening (15) and the intake opening (23) are partially or fully aligned such that liquid may be received into the intake opening (23) from the base opening (15) and discharged through the outlet tube (21);
and
in the closed configuration the base opening (15) and the intake opening (23) are not aligned such that discharge of the liquid received through the base opening (15) is prevented.

9. An outlet valve (1) as claimed in claim 8 wherein the intake opening (23) is arranged in a curved lateral wall of the outlet element (20).

10. An outlet valve (1) as claimed in claim 8 or claim 9 wherein the outlet element (20) further comprises an intake tube (22), the intake tube (22) and the outlet tube (21) together defining a continuous hollow bore for passage of the liquid from the intake opening (23) to an outlet opening (24) arranged at an end of the outlet tube (21) distal to the intake tube (22), wherein a housing (13) protrudes from an outer face (18) of the valve base (10), the housing (13) defining a cylindrical bore (14), a portion of the intake tube (22) being rotatably mounted with the cylindrical bore (14) and wherein the detent (19) is arranged on the outer face (18) of the valve base (10) adjacent to the housing (13) and the one or more protrusions (26, 27) are arranged on the intake tube (22) adjacent to the housing (13).

11. An outlet valve (1) as claimed in any preceding claim wherein the detent (19) is formed of a material which is less rigid than a material forming the one or more protrusions (26, 27).

12. An outlet valve (1) as claimed in any preceding claim wherein the detent (19) is integrally formed with the valve base (10), and wherein optionally the valve base (10) and the detent (19) are formed as a single moulding.

13. An outlet valve (1) as claimed in any preceding claim wherein the one or more protrusions (26, 27) are integrally formed with the outlet element (20), and wherein optionally the outlet element (20) and the one or more protrusions (26, 27) are formed as a single moulding.

14. An ostomy appliance comprising:
an outlet valve (1) as claimed in any preceding claim; and
an ostomy bag (30)
wherein the outlet valve (1) is attached to an outer face (18) of the ostomy bag (30).

15. An ostomy appliance as claimed in claim 14 wherein:
in the closed configuration the outlet element (20) substantially overlies a cavity of the ostomy bag (30); and
in the open configuration at least a portion of the outlet element (20) extends across a lower edge of the cavity of the ostomy bag (30).

## Patentansprüche

1. Ein Auslassventil (1) für einen Stomabeutel (30), umfassend: einen Ventilboden (10) zur Befestigung an einer Fläche des Stomabeutels, wobei der Ventilboden (10) eine Bodenöffnung (15) zur Aufnahme eines Flüssigkeitsaustritts aus dem Stomabeutel (30) definiert; und ein Auslasselement (20), das dazu ausgebildet ist, relativ zu dem Ventilboden (10) von einer geschlossenen Konfiguration, in der der Austritt der durch die Bodenöffnung (15) aufgenommenen Flüssigkeit verhindert wird, zu einer offenen Konfiguration, in der der Austritt der durch die Bodenöffnung (15) aufgenommenen Flüssigkeit ermöglicht wird, drehbar zu sein; wobei eines von dem Auslasselement (20) und dem Ventilboden (10) eine Arretierung (19) aufweist und das andere von dem Auslasselement (20) und dem Ventilboden (10) einen oder mehrere Vorsprünge (26, 27) aufweist; und wobei die Arretierung (19) dazu ausgebildet ist, mit dem einen oder den mehreren Vorsprüngen (26, 27) während einer Drehung des Auslasselements (20) in Eingriff zu kommen, sodass die Arretierung (19) einen oder mehrere der Vorsprünge (26, 27) während der Drehung berührt und darüber gleitet, wobei das Auslasselement (20) ein Auslassrohr (21) an seinem distalen Ende aufweist und das Auslasselement (20) drehbar ist, sodass sich das Auslassrohr (21) entlang eines Drehbogens bewegt, und wobei sich das Auslasselement (20) an einem ersten Ende des Drehbogens in der geschlossenen Konfiguration befindet und sich das Auslasselement (20) an einem zweiten Ende des Drehbogens in der offenen Konfiguration befindet, **dadurch gekennzeichnet, dass** die Arretierung (19) dazu ausgebildet ist, während der Drehung des Auslasselements (20) an einem Punkt, der nicht dem ersten Ende des Drehbogens entspricht, in mindestens einen Vorsprung (26) einzugreifen.

2. Auslassventil (1) nach Anspruch 1, wobei eines von dem Auslasselement (20) und dem Ventilboden (10) die Arretierung (19) aufweist und das andere von dem Auslasselement (20) und dem Ventilboden (10) zwei oder mehr Vorsprünge (26, 27) aufweist.

3. Auslassventil (1) nach Anspruch 2, wobei der Ventilboden (10) die Arretierung (19) aufweist und das Auslasselement (20) die zwei oder mehr Vorsprünge (26, 27) aufweist.

4. Auslassventil (1) nach einem der vorhergehenden Ansprüche, wobei die Arretierung (19) ferner dazu ausgebildet ist, während der Drehung des Auslasselements (20) an einem Punkt in mindestens einen Vorsprung (26) einzugreifen, der dem zweiten Ende des Drehbogens entspricht.

5. Auslassventil (1) nach einem der vorhergehenden Ansprüche, wobei die Arretierung (19) dazu ausgebildet ist, mit zumindest einem Vorsprung (26) in Eingriff zu kommen, wenn sich das Auslasselement (20) an oder nahe einer Grenzposition befindet, wobei die Grenzposition als ein Punkt auf dem Drehbogen definiert ist, an dem das Auslasselement (20) am weitesten von dem ersten Ende entfernt ist und sich auch noch in der geschlossenen Konfiguration befindet.

6. Auslassventil (1) nach einem der vorhergehenden Ansprüche, wobei eine Grenzposition des Auslassrohrs (21) an einem Punkt auf dem Drehbogen definiert ist, der am weitesten von dem ersten Ende entfernt ist, an dem sich das Auslasselement (20) noch in der geschlossenen Konfiguration befindet; und wobei ein Vorsprung (26) des einen oder der mehreren Vorsprünge (26, 27) derart angeordnet ist, dass der Vorsprung (26) die Arretierung (19) berührt, wenn sich das Auslasselement (20) während der Drehung an oder nahe der Grenzposition befindet.

7. Auslassventil (1) nach Anspruch 6, wobei das Auslasselement (20) derart ausgebildet ist, dass das Auslassrohr (21) in der Grenzposition im Gebrauch im Wesentlichen nach oben und in einem spitzen Winkel zu dem Ventilboden (10) abgewinkelt ist.

8. Auslassventil (1) nach einem der Ansprüche 6 bis 7, wobei das Auslasselement (20) eine Einlassöffnung (23) in Fluidverbindung mit dem Auslassrohr (21) definiert, wobei: in der offenen Konfiguration die Bodenöffnung (15) und die Einlassöffnung (23) teilweise oder vollständig fluchtend ausgerichtet sind, sodass Flüssigkeit von der Bodenöffnung (15) in die Einlassöffnung (23) aufgenommen und durch das Auslassrohr (21) abgeleitet werden kann; und in der geschlossenen Konfiguration die Bodenöffnung (15) und die Einlassöffnung (23) nicht fluchtend ausgerichtet sind, sodass ein Ableiten der durch die Bodenöffnung (15) aufgenommenen Flüssigkeit verhindert ist.

9. Auslassventil (1) nach Anspruch 8, wobei die Einlassöffnung (23) in einer gekrümmten Seitenwand des Auslasselementes (20) angeordnet ist.

10. Auslassventil (1) nach Anspruch 8 oder 9, wobei das Auslasselement (20) ferner ein Einlassrohr (22) umfasst, wobei das Einlassrohr (22) und das Auslassrohr (21) zusammen eine durchgehende Hohlbohrung für den Durchgang der Flüssigkeit von der Einlassöffnung (23) zu einer Auslassöffnung (24) definieren, die an einem Ende des Auslassrohrs (21) distal zum Einlassrohr (22) angeordnet ist, wobei ein Gehäuse (13) von einer Außenfläche (18) des Ventilbodens (10) vorsteht, wobei das Gehäuse (13) eine zylindrische Bohrung (14) definiert, wobei ein Abschnitt des Einlassrohrs (22) drehbar mit der zylindrischen Bohrung (14) montiert ist, und wobei die Arretierung (19) auf der Außenfläche (18) des Ventilbodens (10) neben dem Gehäuse (13) angeordnet ist und der eine oder die mehreren Vorsprünge (26, 27) auf dem Einlassrohr (22) neben dem Gehäuse (13) angeordnet sind.

11. Auslassventil (1) nach einem der vorhergehenden Ansprüche, wobei die Arretierung (19) aus einem Material gebildet ist, das weniger steif ist als ein Material, das den einen oder die mehreren Vorsprünge (26, 27) bildet.

12. Auslassventil (1) nach einem der vorhergehenden Ansprüche, wobei die Arretierung (19) einstückig mit dem Ventilboden (10) ausgebildet ist, und wobei optional der Ventilboden (10) und die Arretierung (19) als ein einziges Formteil ausgebildet sind.

13. Auslassventil (1) nach einem der vorhergehenden Ansprüche, wobei der eine oder die mehreren Vorsprünge (26, 27) einstückig mit dem Auslasselement (20) ausgebildet sind, und wobei optional das Auslasselement (20) und der eine oder die mehreren Vorsprünge (26, 27) als ein einziges Formteil ausgebildet sind.

14. Stomavorrichtung, aufweisend: ein Auslassventil (1) nach einem der vorhergehenden Ansprüche; und einen Stomabeutel (30), wobei das Auslassventil (1) an einer Außenfläche (18) des Stomabeutels (30) angeordnet ist.

15. Stomavorrichtung nach Anspruch 14, wobei: in der geschlossenen Konfiguration das Auslasselement (20) im Wesentlichen über einem Hohlraum des Stomabeutels (30) liegt; und in der offenen Konfiguration zumindest ein Teil des Auslasselements (20) sich über einen unteren Rand des Hohlraums des Stomabeutels (30) erstreckt.

## Revendications

1. Soupape (1) de sortie d'un sac (30) de stomie comprenant :
une base (10) de soupape à fixer à une face de l'instrument de stomie, la base (10) de la soupape définissant une ouverture (15) de base de réception d'un liquide sorti du sac (30) de stomie ; et un élément (20) de sortie configuré pour pouvoir tourner par rapport à la base (10) de la soupape d'une configuration fermée, dans laquelle l'évacuation du liquide reçu par l'ouverture (15) de la base est empêchée, à une configuration ouverte, dans laquelle une évacuation du liquide reçu par l'ouverture (15) de la base est rendue possible ; dans laquelle l'un de l'élément (20) de sortie et de la base (10) de la soupape comprend un arrêt (19) et l'autre de l'élément (20) de sortie et de la base (10) de la soupape comprend une plusieurs saillies (26, 27) ; et
dans laquelle l'arrêt (19) est configuré pour s'encliqueter avec la une ou les plusieurs saillies (26, 27) pendant une rotation de l'élément (20) de sortie, de manière à ce que l'arrêt (19) contacte et passe sur une ou plusieurs des saillies (26, 27) pendant la rotation, dans laquelle l'élément (20) de sortie comprend un tube (21) de sortie à son extrémité distale et l'élément (20) de sortie peut tourner de manière à ce que le tube (21) de sortie se déplace suivant un arc de rotation, et dans laquelle, à une première extrémité de l'arc de rotation, l'élément (20) de sortie est dans la configuration fermée et, à une deuxième extrémité de l'arc de rotation, l'élément (20) de sortie est dans la configuration ouverte, **caractérisée en ce que** l'arrêt (19) est configuré pour s'encliqueter avec au moins une saillie (26) en un point pendant une rotation de l'élément (20) de sortie, qui ne correspond pas à la première extrémité de l'arc de rotation.

2. Soupape (1) de sortie suivant la revendication 1, dans laquelle l'un de l'élément (20) de sortie et de la base (10) de la soupape comprend l'arrêt (19) et l'autre de l'élément (20) de sortie et la base (10) de la soupape comprend deux ou plusieurs saillies (26, 27).

3. Soupape (1) de sortie suivant la revendication 2, dans laquelle la base (10) de la soupape comprend l'arrêt (19) et l'élément (20) de sortie comprend les deux ou plusieurs saillies (26, 27).

4. Soupape (1) de sortie suivant l'une quelconque des revendications précédentes, dans laquelle l'arrêt (19) est configuré en outre pour s'encliqueter avec au moins une saillie (26) en un point pendant une rotation de l'élément (20) de sortie, qui correspond à la deuxième extrémité de l'arc de rotation.

5. Soupape (1) de sortie suivant l'une quelconque des revendications précédentes, dans laquelle l'arrêt (19) est configuré pour s'encliqueter avec au moins une saillie (26), lorsque l'élément (20) d'arrêt à une position limite ou proche d'une position limite, dans laquelle la position limite est définie comme un point sur l'arc de rotation où l'élément (20) de sortie est le plus loin de la première extrémité et également encore dans la configuration fermée.

6. Soupape (1) de sortie suivant l'une quelconque des revendications précédentes,
dans laquelle une position limite du tube (21) de sortie est définie en un point sur l'arc de rotation le plus loin de la première extrémité où l'élément (20) est encore dans la configuration fermée ; et
dans laquelle une saillie (26) parmi l'une ou les plusieurs saillies (26, 27) est disposée de manière à ce que la saillie (26) entre en contact avec l'arrêt (19), lorsque l'élément (20) de sortie est à la position limite ou près de la position limite pendant une rotation.

7. Soupape (1) de sortie suivant la revendication 6, dans laquelle l'élément (20) de sortie est configuré, de manière à ce que dans la position limite, en utilisation, le tube (21) de sortie face un angle de manière générale vers le haut et face un angle aigu avec la base (10) de la soupape.

8. Soupape (1) de sortie suivant l'une quelconque des revendications 6 à 7, dans laquelle l'élément (20) de sortie définit une ouverture (23) d'admission en connexion de fluide avec le tube (21) de sortie, dans laquelle :
dans le configuration ouverte, l'ouverture (15) de la base et l'ouverture (23) d'admission sont alignées partiellement ou complètement, de manière à ce que du liquide puisse être reçu dans l'ouverture (23) d'admission, à partir de l'ouverture (15) de la base, et évacué par le tube (21) de sortie ; et
dans la configuration fermée, l'ouverture (15) de la base et l'ouverture (23) d'admission ne sont pas alignées, de manière à empêcher une évacuation du liquide reçu par l'ouverture (15) de la base.

9. Soupape (1) de sortie suivant la revendication 8, dans laquelle l'ouverture (23) d'admission est disposée dans une paroi latérale incurvée de l'élément (20) de sortie.

10. Soupape (1) de sortie suivant la revendication 8 ou la revendication 9, dans laquelle l'élément (20) de sortie comprend en outre un tube (22) d'admission, le tube (22) d'admission et le tube (21) de sortie définissent ensemble un trou creux connu de passage du liquide de l'ouverture (23) d'admission à une ouverture (24) de sortie disposée à une extrémité du tube (21) de sortie, distale du tube (22) d'admission, dans laquelle un boîtier (13) fait saillie d'une face (18) extérieure de la base (10) de la soupape, le boîtier (13) définissant un trou (14) cylindrique, une partie du tube (22) d'admission étant montée tournante avec le trou (14) cylindrique, et dans laquelle l'arrêt (19) est disposé sur la face (18) extérieure de la base (10) de la soupape au voisinage du boîtier (13) et la une ou les plusieurs saillies (26, 27) sur le tube (22) d'admission au voisinage du boîtier (13).

11. Soupape (1) de sortie suivant l'une quelconque des revendications précédentes, dans laquelle l'arrêt (19) est en un matériau, qui est moins rigide qu'un matériau formant la une ou les plusieurs saillies (26, 27).

12. Soupape (1) de sortie suivant l'une quelconque des revendications précédentes, dans laquelle l'arrêt (19) est formé intégralement avec la base (10) de la soupape, et dans laquelle éventuellement la base (10) de la soupape et l'arrêt (19) sont sous la forme d'un seul moulage.

13. Soupape (1) de sortie suivant l'une quelconque des revendications précédentes, dans laquelle la une ou les plusieurs saillies (26, 27) sont formées intégralement avec l'élément (20) de sortie, et dans laquelle éventuellement l'élément (20) de sortie et la une ou les plusieurs saillies (26, 27) sont sous la forme d'un seul moulage.

14. Instrument de stomie comprenant :
une soupape (1) de sortie telle que revendiquée dans l'une quelconque des revendications précédentes ;
et
un sac (30) de stomie
dans lequel la soupape (1) de sortie est fixée à une face (18) extérieure du sac (30) de stomie.

15. Instrument de stomie suivant la revendication 14, dans lequel :
dans la configuration fermée, l'élément (20) de sortie surmonte sensiblement une cavité du sac (30) de stomie ;
et
dans la configuration ouverte, au moins une partie de l'élément (20) de sortie s'étend sur un bord inférieur de la cavité du sac (30) de stomie.
